(19) Europäisches Patentamt
European Patent Office
Office européen des brevets

(11) EP 0 825 229 B1

(12) **EUROPÄISCHE PATENTSCHRIFT**

(45) Veröffentlichungstag und Bekanntmachung des Hinweises auf die Patenterteilung:
**12.05.2004 Patentblatt 2004/20**

(51) Int Cl.7: **C08L 75/08**, C09D 7/00

(21) Anmeldenummer: **97113524.9**

(22) Anmeldetag: **06.08.1997**

(54) **Verdickungsmittel-Zubereitungen auf Polyurethanbasis und Verwendung zur Verdickung wässriger Systeme**

Polyurethane based thickening compositions and their use to thicken aqueous systems

Préparation de liants à base de polyuréthane et leur utilisation comme liant pour des systèmes aqueux

(84) Benannte Vertragsstaaten:
**BE DE ES FR GB IT NL SE**

(30) Priorität: **19.08.1996 DE 19633195**

(43) Veröffentlichungstag der Anmeldung:
**25.02.1998 Patentblatt 1998/09**

(73) Patentinhaber: **BAYER AG**
**51368 Leverkusen (DE)**

(72) Erfinder:
- **Mazanek, Jan, Dr.**
**51061 Köln (DE)**
- **Kober, Hermann**
**51467 Bergisch Gladbach (DE)**
- **Walz, Klaus, Dr.**
**51381 Leverkusen (DE)**

(56) Entgegenhaltungen:
**EP-A- 0 307 775**
**EP-A- 0 618 243**
**DE-A- 4 333 106**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 825 229 B1

Printed by Jouve, 75001 PARIS (FR)

## Beschreibung

Die Erfindung betrifft eine neue Verdickungsmittel-Zubereitung zur Verdickung wässriger Systeme, die sich durch eine besonders niedrige Eigenviskosität bei gleichzeitig hoher Verdickungswirkung auszeichnet sowie ihre Verwendung.

Verdickungsmittel auf Polyurethanbasis für wässrige Systeme werden in zahlreichen Veröffentlichungen beschrieben (vgl. z.B. DE-A 1 444 243, DE-A 3 630 319, EP-A-0 031 777, EP-A-0 307 775, EP-A-0495 373, US-A 4 079 028, US-A 4 155 892, US-A 4 499 233 oder US-A 5 023 309).

Diesen Verdickungsmitteln des Standes der Technik gemeinsam ist das gleichzeitige Vorliegen von (i) hydrophilen Segmenten in einer Menge von mindestens 50 Gew.-%, (ii) hydrophoben Segmenten in einer Menge von maximal 10 Gew.-% und (iii) Urethangruppen. Unter "hydrophilen Segmenten" sind hierbei insbesondere Polyetherketten mit mindestens 5 Kettengliedern zu verstehen, deren AlkylenoxidEinheiten zumindest zu 60 Mol-% aus Ethylenoxideinheiten bestehen. Unter "hydrophoben Segmenten" sind hierbei insbesondere Kohlenwasserstoff-Segmente mit mindestens 6 Kohlenstoffatomen zu verstehen.

Die nachstehend genannte Komponente a) der erfindungsgemäßen Zubereitungen entspricht vorzugsweise dieser Definition.

Diese Polyurethanverdicker eignen sich als Hilfsmittel für die Einstellung von rheologischen Eigenschaften von wässrigen Systemen wie Auto- und Industrielacken, Putz- und Anstrichfarben, Druck- und Textilfarben, Pigmentdruckpasten, pharmazeutischen, kosmetischen Zubereitungen, Pflanzenschutzformulierungen oder Füllstoffdispersionen.

Obwohl die bekannten Polyurethanverdicker eine breite Anwendung finden, weisen sie einen wesentlichen Nachteil auf: Sie besitzen in Form ihrer wässrigen Lösung eine zu hohe Eigenviskosität, die ihre Einarbeitung in die wässrigen Systeme sehr erschwert.

In der Vergangenheit wurden viele Versuche unternommen, die Eigenviskosität der Verdickungsmittel herabzusetzen. Bereits bei der Herstellung der Verdicker wurde versucht, zum Beispiel durch Erniedrigung des Molekulargewichts die Eigenviskosität zu erniedrigen. Dies hat aber innerhalb einer homologen Reihe eine Verschlechterung der Verdickerwirkung zur Folge.

Der naheliegende Gedanke, die Viskosität der wässrigen Lösungen durch Verdünnen mit Wasser herabzusetzen, ist selbstverständlich mit einer nachteilhaften Abnahme der Konzentration des Wirkstoffs und damit bei gleicher Gesamtdosierung mit Erniedrigung der verdickenden Wirkung verbunden.

Eine gängige Methode, die Eigenviskosität der wässrigen Polyurethanverdickerlösungen herabzusetzen, ist der Zusatz von wassermischbaren Lösungsmitteln wie zum Beispiel wasserlöslichen ein- oder mehrwertigen Alkoholen. Der wesentliche Nachteil dieser an sich sehr wirksamen Methode liegt in der Verschlechterung der Umweltverträglichkeit der wässrigen Zubereitungen. Dazu kommt, dass die Menge an Lösemitteln, die zum Erreichen einer gewünschten Viskosität zugesetzt werden müssen, oft relativ hoch ist. Größere Anteile an Lösemitteln können auch zu einer Verschlechterung der anwendungstechnischen Eigenschaften der wässrigen Zubereitungen führen, wie zum Beispiel der Streicheigenschaften oder der Stabilität.

Eine andere Möglichkeit, die Viskosität von wässrigen Lösungen von Polyurethanverdickem herabzusetzen besteht darin, sie mit Emulgatoren wie alkoxylierten Alkoholen oder Phenolen zu versetzen. Diese Methode hat jedoch den Nachteil, dass diese Hilfsmittel in hohen Konzentrationen verwendet werden müssen, um eine ausreichende Herabsetzung der Eigenviskosität des Verdickers zu erreichen. Aber auch mit dieser Maßnahme ließ sich die Eigenviskosität der Verdicker bisher nicht immer und insbesondere nicht bei hochwirksamen Verdickungsmitteln - auf das gewünschte Niveau erniedrigen.

Die der Erfindung zugrundeliegende Aufgabe besteht daher darin, neue Verdickungsmittel-Zubereitungen auf Polyurethanbasis für wässrige Systeme zur Verfügung zu stellen, die in Form ihrer wässrigen Lösungen oder Dispersionen im Vergleich zu analogen Systemen des Standes der Technik bei zumindest gleich guter Verdickerwirkung eine deutlich erniedrigte Eigenviskosität aufweisen.

Diese Aufgabe konnte überraschend dadurch gelöst werden, dass bei der Formulierung der wässrigen PUR-Verdickerzubereitungen eine Kombination aus (teil)aromatischen nichtionischen Tensiden und als Komponente c) die näher beschriebenen Verbindungen verwendet wurden.

Gegenstand der Erfindung ist demzufolge eine Verdickungsmittel-Zubereitung zur Verdickung wässriger Systeme, bestehend aus einem Gemisch aus

a) einem Urethangruppen aufweisenden, in Wasser löslichen oder dispergierbaren Verdickungsmittel,

b) einem nichtionischen (gegebenenfalls alk)aromatischen Emulgator und

c) mindestens einem weiteren Zusatzmittel und gegebenenfalls

d) weiteren Hilfsmitteln

dadurch gekennzeichnet, dass die Komponente c) aus mindestens einer Verbindung besteht, die durch Umsetzung von 6 bis 22 C-Atome aliphatischen, aktive Wasserstoffatome enthaltenden Verbindungen mit $C_2$-$C_4$-Alkoxiden entsteht.

Gegenstand der Erfindung ist auch die Verwendung dieser Verdickungsmittel-Zubereitung in wässrigen Systemen, ausgewählt aus der Gruppe bestehend aus wässrigen Kraftfahrzeug- und Industrielacken, Putz- und Anstrichfarben, Druck- und Textilfarben, Pigmentdruckpasten, wässrigen pharmazeutischen, kosmetischen Formulierungen, Pflanzenschutzformulierungen, Füllstoff- und Pigmentdispersionen, Zubereitungen von Waschmitteln, Klebstoffen, Wachsen und Polituren sowie für die Erdölförderung.

Die Komponente a) der erfindungsgemäßen Zubereitungen besteht aus Polyurethanverdickungsmitteln der an sich bekannten Art mit dem obengenannten Mindestgehalt an hydrophilen bzw. hydrophoben Segmenten und Urethangruppen.

Bei der Komponente b) handelt es sich vorzugsweise um mindestens eine Verbindung der Formel (II)

$$R\text{-}[O\text{-}(Q')_x\text{-}H]_y \qquad (II),$$

in welcher

R für einen gegebenenfalls mehrere aromatische Ringe umfassenden und/oder inerte Substituenten aufweisenden aromatischen und/oder alkaromatischen Kohlenwasserstoffrest mit 6 bis 50 Kohlenstoffatomen, steht,

Q' für Alkylenoxideinheiten steht, wie sie bei der Alkoxylierung von Hydroxylgruppen enthaltenden Startermolekülen mit $C_2$-$C_4$-Alkylenoxiden entstehen, wobei Q' vorzugsweise für Ethylenoxid- und/oder Propylenoxid-Einheiten steht,

x für eine Zahl von 1 bis 300, vorzugsweise 5 bis 100 und besonders bevorzugt 10 bis 30 steht und

y für eine Zahl von 1 bis 20, vorzugsweise 1 bis 10 und besonders bevorzugt 1 bis 4 steht.

Als Komponente c) werden Verbindungen der allgemeinen Formel (III)

$$R_2\text{-}[EO_x\text{-}PO_y]\text{-}H \qquad (III),$$

$R_2$ für einen linearen Rest mit 6 bis 15 Kohlenwasserstoffatomen steht, EO, PO Ethylenoxid- bzw. Propylenoxidreste bedeuten,

x für eine Zahl von 3 bis 10

y für eine Zahl von 0 bis 6

eingesetzt.

Es handelt sich bei den Komponenten c) um an sich bekannte Alkylierungsprodukte von geeigneten Startermolekülen, wobei als Alkylenoxide insbesondere Ethylenoxid, Propylenoxid oder die isomeren Butylenoxide in Betracht kommen. Vorzugsweise handelt es sich bei den Alkylenoxiden jedoch um Ethylenoxid oder um Gemische aus Ethylenoxid mit. Grundsätzlich möglich ist die Verwendung unterschiedlicher Alkylenoxide nacheinander, so dass unterschiedliche Polyetherblöcke entstehen.

Geeignete Startermoleküle sind insbesondere der obengemachten Definition von R, X und y entsprechende mono- und polyfunktionelle Phenole

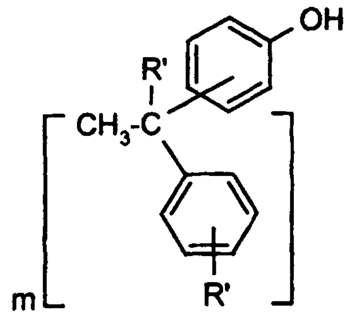

m = 0,5 bis 2,8
R' = H, Methyl

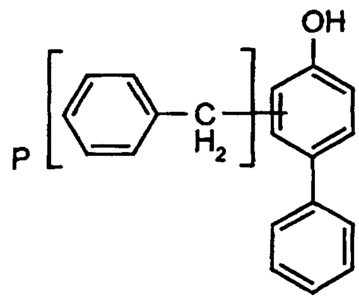

p = 0,5 bis 2,8
(m und p sind statistische Mittelwerte)

Als Komponente c) werden vorzugsweise Verbindungen der Formel

$$R_2\text{-}[(Q_2\text{-})_xH]_y \qquad (I)$$

worin

- $R_2$ für einen gegebenenfalls verzweigten, und/oder ungesättigten aliphatischen Rest mit 6 bis 22, bevorzugt 8 bis 16, besonders bevorzugt 8 bis 12 Kohlenstoffatomen,
  und/oder für einen cycloaliphatischen Rest mit 6 bis 10 Kohlenstoffatomen
  und/oder für einen heterocyclischen Rest mit 5 bis 12, bevorzugt 5 bis 7 Ringatomen steht,
  so wie er durch Abtrennung des aktiven Wasserstoffs von entsprechenden Alkoholen, Aminen, Carbonsäuren und/oder Amiden entsteht
- $Q_2$ für Alkylenoxideinheiten steht, wie sie bei der Alkoxylierung von Hydroxylgruppen enthaltenden Startermolekülen mit $C_2$-$C_4$-Alkylenoxiden entstehen, wobei $Q_2$ vorzugsweise für Ethylenoxid- und/oder Propylenoxid-Einheiten steht,
- x für eine Zahl von 1 bis 30, vorzugsweise 2 bis 20 und besonders bevorzugt 4 bis 10 steht und
- y für eine Zahl von 1 bis 10, vorzugsweise 1 bis 6 und besonders bevorzugt 1 und 2 steht.

Es handelt sich bei den Komponenten c) um an sich bekannte Alkoxylierungsprodukte von geeigneten Startermolekülen, wobei als Alkylenoxide insbesondere Ethylenoxid, Propylenoxid oder die isomeren Butylenoxide in Betracht kommen. Vorzugsweise handelt es sich bei den Alkylenoxiden jedoch um Ethylenoxid oder um Gemische von

Ethylenoxid mit Propylenoxid. Grundsätzlich möglich ist die Verwendung unterschiedlicher Alkylenoxide nacheinander, so dass unterschiedliche Polyetherblöcke entstehen.

Geeignete Startermoleküle für die Komponente c) sind beispielsweise: n-Hexanol, n-Octanol, Isooctanol, n-Nonanol, Isononanol, n-Decanol, iso-Undecanol, Undecanol, n-Dodecanol, Tetradecanol, Hexadecanol sowie deren Gemische, so wie sie beispielsweise bei technischen Synthesen oder aus Naturprodukten gewonnen werden. Weitere Beispiele: Cyclohexanol, Methylcyclohexanol, Hydroxytetralin, n-Hexylamin, n-Octylamin, n-Dodecylamin, Dodecansäureamid, Verbindungen der Formel

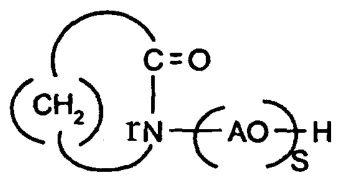

worin

r eine Zahl von 2 bis 20 bedeutet,

AO einen Alkylenoxidrest mit 2 bis 3 Kohlenstoffatomen bedeutet und

S eine Zahl von 1 bis 30 bedeutet.

Weitere, gegebenenfalls mitverwendete Hilfsmittel d) sind beispielsweise mehrwertige Alkohole wie beispielsweise Propylenglykol ggf. in Gemischen mit Wasser, die u.a. zur Formulierung der Einzelkomponenten dienen können.

In den erfindungsgemäßen Verdickungsmittel-Zubereitungen liegt die Komponente b) in einer Menge von 0,5 bis 80, vorzugsweise 5 bis 50 und besonders bevorzugt 10 bis 30 Gew.-%, bezogen auf Gesamtgemisch, die Komponente c) in einer Menge von 0,5 bis 80, vorzugsweise 1 bis 50 und besonders bevorzugt 1 bis 40 Gew.-%, bezogen auf Gesamtgemisch vor, wobei die Gesamtmenge der Komponenten b) und c) bei maximal 90, vorzugsweise maximal 70 Gew.-% besonders bevorzugt maximal 50 %, bezogen auf Gesamtgemisch, beträgt. Unter "Gesamtgemisch" ist hierbei die Gesamtheit der wasserfreien Einzelkomponenten a), b) und c) zu verstehen.

Neben diesen erfindungswesentlichen Einzelkomponenten können, wie bereits ausgeführt, noch weitere Hilfsmittel d) vorliegen. Der Gewichtsanteil dieser Hilfsmittel liegt jedoch bei maximal 30 Gew.-%, bezogen auf das Gewicht des oben definierten "Gesamtgemisches".

Die Herstellung der erfindungsgemäßen Zubereitungen kann in an sich bekannter Weise erfolgen. So können beispielsweise die Komponenten b) und c) nacheinander unter Rühren und gegebenenfalls Erwärmen zu dem gegebenenfalls in Wasser gelösten Polyurethanverdicker a) zugegeben werden; ebenfalls möglich ist die Herstellung einer Primärmischung aus den Komponenten b) und c), die anschließend zum gegebenenfalls in Wasser gelösten Polyurethanverdicker a) zugegeben wird. In diesem Zusammenhang kommt beispielsweise die Mitverwendung von üblichen Lösungs- bzw. Verdünnungsmitteln als Komponente d) zwecks Verbesserung der Mischbarkeit der Einzelkomponenten in Betracht.

Eine weitere Variante der Herstellung der erfindungsgemäßen Zubereitungen besteht in der Zugabe der Komponenten b) und c), sowie gegebenenfalls von Wasser zum Polyurethanverdicker a) unmittelbar nach dessen Herstellung. Diese Methode ist besonders bevorzugt, weil sie wirtschaftliche Vorteile gegenüber den vorstehend genannten Methoden bietet.

Die gebrauchsfertigen erfindungsgemäßen Zubereitungen stellen in der Regel wässrige Lösungen oder Dispersionen mit einem Feststoffgehalt von 10 bis 90, vorzugsweise 30 bis 70 und besonders bevorzugt 40 bis 50 Gew.-% dar, wobei unter "Feststoff" das oben definierte "Gesamtgemisch" zuzüglich gegebenenfalls vorliegenden weiteren Komponenten d) zu verstehen ist.

Die Beurteilung der Eigenviskosität der erfindungsgemäßen Zubereitung kann nach bekannten Methoden erfolgen, wie z.B. im Haake-Rotationsviskosimeter VT 500 oder im Brookfield-Viskosimeter. Die Viskosität kann in breiten Grenzen variieren. Vorzugsweise besitzen sie aber Fließeigenschaften, die eine problemlose Handhabung durch Gießen oder Pumpen ermöglichen. Sie liegt, gemessen bei $10{,}3 s^{-1}$ und 23°C, bei 100 bis 60.000 mPa.s, bevorzugt bei 100 bis 20.000 mPa.s, besonders bevorzugt bei 100 bis 10.000 mPa.s.

Die erfindungsgemäßen Zubereitungen können aufgrund ihrer relativ niedrigen Eigenviskosität auch in ihrer konzentrierten Form der erfindungsgemäßen Verwendung zugeführt werden. Besonders bemerkenswert in diesem Zusammenhang ist die Beobachtung, dass die Verdickerwirkung in den erfindungsgemäßen Zubereitungen trotz der

vergleichsweise deutlich erniedrigten Eigenviskosität der erfindungsgemäßen Verdickungsmittel nicht oder nur unwesentlich vermindert wird.

Ein weiterer Vorteil der erfindungsgemäßen Zubereitungen liegt in ihrer Verträglichkeit mit den zu verdickenden wässrigen Systemen, z.B. Dispersionsfarben, was eine leichte Einarbeitbarkeit der Verdickungsmittel ermöglicht, wobei gleichzeitig die sogenannte Reifezeit der erhaltenen verdickten Zubereitungen, d.h. die Zeit bis zum Erreichen der maximal möglichen Viskosität, in der Regel wesentlich verkürzt wird.

Die erfindungsgemäßen Zubereitungen eignen sich zur Verdickung von wässrigen bzw. überwiegend wässrigen Systemen wie Farben, Druck- und Pigmentpasten, Füllstoffund Pigmentdispersionen, Textil-, Leder- und Papierhilfsmittel, Zubereitung für die Erdölförderung, Zubereitungen von Waschmittel, Klebstoffen, Wachsen, für Polituren, Formulierungen für pharmazeutische und veterinäre Zwecke, Pflanzenschutzzubereitungen oder kosmetische Artikel. Auch das Wasser selbst kann mit den erfindungsgemäßen Polyurethanverdickern angedickt werden, um dann gegebenenfalls mit weiteren Zusätzen versetzt zu werden oder selbst zu wässrigen Zubereitungen zugesetzt werden.

Die erfindungsgemäßen Verdickungsmittel-Zubereitungen eignen sich nicht nur zur Verdickung von rein wässrigen Systemen, sondern auch von solchen Systemen, die anteilmäßig organische Lösungsmittel oder andere flüchtige Zusätze wie beispielsweise mehrwertige Alkohole enthalten. Selbstverständlich können die zu verdickenden wässrigen Systeme die in solchen Systemen üblichen Hilfs- und Zusatzmittel wie Entschäumer, Fließ- und Verlaufhilfsmittel, Füllstoffe und Pigmente enthalten.

Beispiele für wässrige Systeme, die erfindungsgemäß verdickt werden können, sind wässrige Polyacrylatdispersionen, wässrige Dispersionen von Mischpolymerisaten olefinisch ungesättigter Monomeren, wässrige Polyvinylacetatdispersionen, wässrige Polyurethandispersionen, wässrige Polyesterdispersionen, 2-Komponenten-Lacke und insbesondere gebrauchsfertige Zubereitungen der oben bereits angesprochenen Art und Basis derartiger Dispersionen.

Im Falle der Verwendung der erfindungsgemäßen Zubereitungen zur Verdickung von Dispersionsfarben führt dies oftmals zu einem verbesserten Verlauf dieser Systeme und zu einer verbesserten Oberflächenbeschaffenheit der aus derartigen Dispersionsfarben hergestellten Filme. Ein weiterer Vorteil der erfindungsgemäßen Zubereitungen liegt in dem Umstand begründet, dass ihr Einsatz in pigmentierten bzw. Füllstoffe enthaltenden Dispersionsfarben oftmals zu einer verbesserten Benetzbarkeit dieser Festkörper führt, wodurch der Dispergierprozeß, d.h. die Herstellung der gebrauchsfertigen Dispersionsfarben erleichtert wird. Lackfilme, die unter Verwendung von erfindungsgemäß verdickten Dispersionsfarben hergestellt werden, zeichnen sich im übrigen durch einen verbesserten Glanz aus.

## <u>Beispiele 1 bis 5</u>

Es wurde ein Verdickungsmittel gemäß Beispiel 5, DE-A 4 327 481 eingesetzt.

Zu 26 g des so hergestellten Polyurethanverdickers a) wurde als Komponente b) verschiedene Mengen eines nichtionischen Tensids der Formel

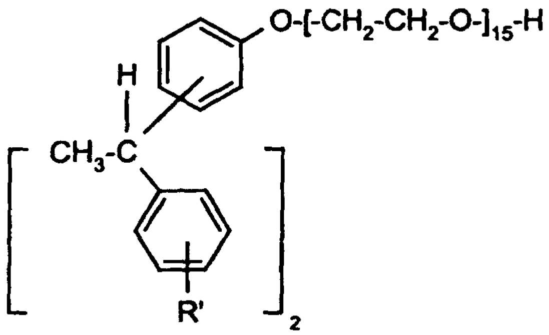

sowie als Komponente c) verschiedene Mengen eines aus einem 1:1-Gemisch aus Nonyl- und Undecylalkohol ($R_{9-11}$) und Propylenoxid (PO) und Ethylenoxid (EO) hergestellten niedermolekularen Polyethers $R_{9-11}$-$EO_6$-$PO_{2,5}$ sowie Wasser (auf 100 g) zugegeben. Die Gemische wurden 30 Minuten bei 70°C gerührt (500 U/min), anschließend 8 Stunden bei 50°C und dann 8 Stunden bei Raumtemperatur gelagert. Die Viskosität der so erhaltenen Lösungen wurden im Haake-Viskosimeter VT 500, Meßkörper SV DIN, bei 23°C und 10,3 $s^{-1}$ gemessen und sind in Tabelle 1 aufgeführt.

Tabelle 1

| Zusammensetzung der Verdickungsmittel-Zubereitung (Gew.-%, Rest Wasser) | | | | |
|---|---|---|---|---|
| Beispiel Nr. | Verdicker a) | Komponente b) | Komponente c) | Viskosität (mPa.s/ 23°C) |
| 1 | 26 | 19 | 5 | 13850 |
| 2 | 26 | 14 | 10 | 10400 |
| 3 | 26 | 9 | 15 | 6800 |
| 4 | 26 | 7 | 17 | 5400 |
| 5 | 26 | 4 | 20 | 5100 |

**Beispiele 6 bis 10**

Es wurde wie in Beispiel 1 bis 5 gearbeitet, jedoch wurden die Komponenten b) und c) nicht oder nur einzeln zugegeben. Die Ergebnisse sind in Tabelle 2 aufgeführt. Sie zeigen, dass weder die Komponente b noch die Komponente c) alleine ausreicht, um anwendungstechnisch gewünschte niedrigviskose und lagerstabile Produkte zu erhalten.
Entweder ist die Viskosität zu hoch (Beispiel 7), oder die Gemische sind nicht lagerstabil (Beispiele 8 bis 10)

Tabelle 2

| Zusammensetzung der Verdickungsmittel-Zubereitung (Gew.-%, Rest Wasser) | | | | | |
|---|---|---|---|---|---|
| Beispiel Nr. | Verdicker a) | Komponente b) | Komponente c) | Viskosität (mPa.s/23°C) | Warmlagerung 50°C |
| 6 | 26 | - | - | zu hoch[2)] (nicht meßbar) | nicht relevant |
| 7 | 26 | 24 | - | 30500 | in Ordnung |
| 8 | 26 | - | 24 | 4000 | Trennung in 2 Schichten bei > 40°C |
| 9 | 28 | - | 22 | 6300 | |
| 10 | 30 | - | 20 | 11100 | |

2) > 60000 mPa.s

**Beispiele 11 bis 15**

Die nachfolgenden Beispiele zeigen, dass die verdickende Wirkung der Komponente a) durch die viskositätserniedrigenden Zusätze b) und c) nicht negativ beeinflußt wird. Die Werte streuen innerhalb der bekannten Grenzen.

**Messung der Verdickerwirkung**

Zu jeweils 98 g einer handelsüblichen Polyacrylatdispersion (Dilexo® RA3 der Fa. Condea, Hamburg), werden in fünf Parallelversuchen jeweils 2 g einer wässrigen Lösung einer Verdicker-Zubereitung zugegeben. Die Konzentration der Lösungen liegt jeweils bei 2,5 Gew.-%, bezogen auf den Polyurethanverdicker a). Die so hergestellten Gemische werden 5 Minuten bei 2000 U/min. gerührt. Die so erhaltenen homogenen Dispersionen werden 3 h bei 23°C gelagert. Anschließend wird wie oben beschrieben die Viskosität gemessen. Die Ergebnisse sind in Tabelle 3 aufgeführt.

Tabelle 3

| Beispiel Nr. | Verdickungsmittel-Zubereitung aus Beispiel Nr. | Verdickerwirkung Viskosität (mPa.s) (23°C) |
|---|---|---|
| 11 | 7 (ohne Komponente c)) | 12400 |
| 12 | 2 | 12200 |
| 13 | 4 | 12600 |
| 14 | 9 | 12300 |
| 15 | 10 | 12500 |

**Beispiele 16 bis 24**

Es wurde wie im Beispiel 1 beschrieben gearbeitet, jedoch wurden verschiedene Komponenten c) eingesetzt. Das Verhältnis der Komponenten a), b) und c) betrug in allen Fällen 26:12:12
(50 Teile (= Rest)Wasser).

Tabelle 4

| **Zusammensetzung der Verdickungsmittel-Zubereitungen (Gew.-%, Rest Wasser)** | | | |
|---|---|---|---|
| Beispiel Nr. | Zusammensetzung der Komponente c) | Viskosität mPa.s | Verdickerwirkung mPa.s/23°C |
| 16 | Gemisch Nonanol/Undecanol 1:1/7EO | 10900 | 12700 |
| 17 | Tridecanol/4EO/1,5PO | 13800 | 12400 |
| 18 | Tridecanol/5EO/3PO | 12800 | 12500 |
| 19 | Isooctanol/6EO/4PO | 12900 | 12450 |
| 20 | Isooctanol/5EO/5PO | 10400 | 12300 |
| 21 | Nonanol/Undecanol 1:1/5EO/5PO) | 10000 | 12700 |
| 22 | Isodecanol/6EO/5PO | 13100 | 12400 |
| 23 | 2-Ethylhexanol/8PO/6EO | 26300 | 12200 |
| als Vergleich: 24 | 24 Teile Komponente b) ohne Komponente c) | 30500 | 12500 |

**Beispiele 25 bis 34**

Es wurde gearbeitet wie in Beispiel 1 bis 5 beschrieben, jedoch wurde als Verdicker a) eine Verbindung analog US-Patentschrift 4 079 028, Beispiel 79, genommen jedoch mit Hexamethylendiisocyanat anstelle von Toluylendiisocyanat. Die Ergebnisse sind in Tabelle 6 zusammengestellt. In Tabelle 6 sind auch die gemäß Beispielen 11 bis 15 gemessenen Verdickerwirkungen der erfindungsgemäßen Zubereitungen aufgeführt, die zeigen, dass deren Einsatz keinen negativen Einfluß auf die Wirksamkeit der Verdickungsmittel hat. Das Verhältnis der Komponenten a), b) (aus Beispiel 1) und c) betrug in allen Fällen 26:12:12 (Rest Wasser).

Tabelle 5

| **Zusammensetzung der Verdickungsmittel-Zubereitungen (Gew.-%, Rest Wasser)** | | | |
|---|---|---|---|
| Beispiel Nr. | Zusammensetzung der Komponente c) | Viskosität mPa.s | Verdickerwirkung mPa.s/23°C |
| 25 | Nonanol/Undecanol 1:1/7EO | 3300 | 9300 |
| 26 | Tridecanol/4EO/1,5PO | 3900 | 9100 |
| 27 | Tridecanol/5EO/3PO | 3600 | 9000 |

Tabelle 5 (fortgesetzt)

| Zusammensetzung der Verdickungsmittel-Zubereitungen (Gew.-%, Rest Wasser) | | | |
|---|---|---|---|
| Beispiel Nr. | Zusammensetzung der Komponente c) | Viskosität mPa.s | Verdickerwirkung mPa.s/23°C |
| 28 | Isooctanol/6EO/4PO | 3700 | 9400 |
| 29 | Isodecanol/5EO/5PO | 3200 | 9250 |
| 30 | Nonanol/Undecanol 1:1/5EO/5PO | 2800 | 9150 |
| 31 | Isodecanol/6EO/5PO | 5200 | 9100 |
| 32 | 2-Ethylhexanol/8PO/6EO | 6900 | 9300 |
| 33 | Nonanol/Undecanol 1:1/6EO/2,5EO | 2700 | 9200 |
| als Vergleich: 34 | 24 Teile Komponente b) ohne Komponente c) | 8250 | 9200 |

**Beispiel 35 bis 37**

Es wurde wie in den Beispielen 25 bis 33 gearbeitet, jedoch wurden verschiedene Komponenten b) sowie Komponente c) aus Beispiel 1 im Verhältnis 1:1 (jeweils 12 Teile) eingesetzt. Die Ergebnisse sind in Tabelle 6 zusammengestellt.

Tabelle 6

| Zusammensetzung der Verdickungsmittel-Zubereitungen (Gew.-%, Rest Wasser) | | | |
|---|---|---|---|
| Beispiel Nr. | Komponente b) | Viskosität mPa.s | Verdickerwirkung mPa.s/23°C |
| 35 | Borchigen DFN (Aralkylphenol/14EO) | 3000 | 9250 |
| 36 | Komponente c) aus Beispiel 1, jedoch mit 2,2 Mol Styrol/Mol Phenol und 16EO | 3500 | 9300 |
| 37 | Nonylphenol/10EO | 2900 | 9100 |

**Beispiele 38 bis 49**

Es wurde wie im Beispiel 1 gearbeitet, jedoch wurden als Komponente b) die folgenden eingesetzt:

I. Kondensationsprodukt aus 2,2 Mol Benzylchlorid und 1 Mol Hydroxybisphenyl, umgesetzt mit 15 Mol EO

II. Kondensationsprodukt aus 2,8 Mol Styrol und 1 Mol Phenol, umgesetzt mit 17 Mol Ethylenoxid

III. Kondensationsprodukt aus 2 Mol Styrol und 1 Mol Phenol, umgesetzt mit 12 Mol Ethylenoxid

IV. Kondensationsprodukt von 2,8 Mol Vinyltoluol mit 1 Mol Phenol, umgesetzt mit 20 Mol EO

V. Kondensationsprodukt von 1,8 Mol Styrol mit 1 Mol Phenol, umgesetzt mit 14 Mol EO

VI. Kondensationsprodukt von 2 Mol Styrol mit 1 Mol Phenol, umgesetzt mit 20 Mol EO und 4 Mol PO

VII. Kondensationsprodukt von 2 Mol Styrol mit 1 Mol Phenol, umgesetzt mit einer Mischung aus 13 Mol EO und 4 Mol PO

VIII. Kondensationsprodukt von 1,8 Mol α-Methylstyrol und 1 Mol Phenol, umgesetzt mit 16 Mol EO

Diese Verbindungen wurden in Verhältnissen wie in Tabelle 7 aufgeführt, zur Herstellung der erfindungsgemäßen Zubereitungen eingesetzt.

**Tabelle 7**

| Beispiel | Komponente b) | Komponente c) | Viskosität mPa·s | Verdickerwirkung mPa·s/23°C |
|---|---|---|---|---|
| 38 | 12 Tle I | 12 Tle Komponente c) aus Bsp. 1 | 11100 | 12300 |
| 39 | 12 Tle II | 12 Tle n-Hexanol x 4 EO | 12000 | 11800 |
| 40 | 14 Tle I | 10 Tle n-Hexanol x 4 EO | 13600 | 11900 |
| 41 | 10 Tle V | 14 Tle n-Octanol x 4 EO/2 PO | 10500 | 12100 |
| 42 | 14 Tle VI | 10 Tle Caprolactam x 4 PO | 14200 | 12400 |
| 43 | 12 Tle IV | 12 Tle n-Nonanol x 4,5 EO/2,5 PO | 12200 | 12500 |
| 44 | 20 Tle I | 4 Tle n-Hexanol x 3 EO/1 PO | 21600 | 12700 |
| 45 | 13 Tle III | 11 Tle n-Octanol x 4 EO | 12900 | 12200 |
| 46 | 12 Tle VII | 12 Tle n-Hexanol x 5 EO | 11800 | 12500 |
| 47 | 12 Tle VIII | 12 Tle n-Dodecanol x 5 EO/2 PO | 13400 | 12700 |
| 48 | 14 Tle I | 10 Tle n-Hexadecanol x 8 EO/3 PO | 15100 | 12400 |
| 49 (Vergleich) | 24 Teile Komponente b) aus Beispiel 1 | | 30500 | 12500 |

**Patentansprüche**

1. Verdickungsmittel zur Verdickung wässriger Systeme, bestehend aus einem Gemisch aus

   a) einem Urethangruppen aufweisenden, in Wasseröslichen oder dispergierbaren Verdickungsmittel,

   b) einem nichtionischen (gegebenenfalls alk)aromatischen Emulgator und

   c) mindestens einem weiteren Zusatzmittel und gegebenenfalls

   d) weiteren Hilfsmitteln

   **dadurch gekennzeichnet, dass** die Komponente c) aus mindestens einer Verbindung besteht, die durch Umsetzung von 6 - 22 C-Atome aliphatischen, aktive Wasserstoffatome enthaltenden Verbindungen mit $C_2$-$C_4$-Alkoxiden entstehen.

2. Verdickungsmittel nach Anspruch 1, **dadurch gekennzeichnet, dass** als Komponente c) Verbindungen der Formel (I)

$$R_2\text{-}[(Q_2\text{-})_xH]_y \qquad \text{(I)},$$

   eingesetzt werden,
   worin

   $R_2$ für einen gegebenenfalls verzweigten, und/oder ungesättigten aliphatischen Rest mit 6 bis 22 Kohlenstoffatomen,
   und/oder für einen cycloaliphatischen Rest mit 6 bis 10 Kohlenstoffatomen
   und/oder für einen heterocyclischen Rest mit 5 bis 12, steht,
   so wie er durch Abtrennung des aktiven Wasserstoffs von entsprechenden Alkoholen, Aminen, Carbonsäuren und/oder Amiden entsteht

   $Q_2$ für Alkylenoxideinheiten steht, wie sie bei der Alkoxylierung von Hydroxylgruppen enthaltenden Startermolekülen mit $C_2$-$C_4$-Alkylenoxiden entstehen,

   x für eine Zahl von 1 bis 30 steht und

   y für eine Zahl von 1 bis 10 steht.

3. Verdickungsmittel nach Anspruch 1, **dadurch gekennzeichnet, dass** als Komponente b) Verbindung der Formel (II)

$$R\text{-}[O\text{-}(Q')_x\text{-}H]_y \qquad \text{(II)},$$

   in welcher

   R für einen gegebenenfalls mehrere aromatische Ringe umfassenden und/oder inerte Substituenten aufweisenden aromatischen und/oder alkaromatischen Kohlenwasserstoffrest mit 6 bis 50 Kohlenstoffatomen, steht,

   Q' für Alkylenoxideinheiten steht, wie sie bei der Alkoxylierung von Hydroxylgruppen enthaltenden Startermolekülen mit $C_2$-$C_4$-Alkylenoxiden entstehen,

   x für eine Zahl von 1 bis 300 und

   y für eine Zahl von 1 bis 20 steht,

eingesetzt werden.

**4.** Verdickungsmittel nach Anspruch 1, **dadurch kennzeichnet, dass** als Komponente c) Verbindungen der allgemeinen Formel (III)

$$R_2\text{-}[EO_x\text{-}PO_y]\text{-}H \qquad \text{(III)},$$

$R_2$ für einen linearen Rest mit 6 bis 15 Kohlenwasserstoffatomen steht, EO, PO Ethylenoxid- bzw. Propylenoxidreste bedeuten,

x für eine Zahl von 3 bis 10 steht,

y für eine Zahl von 0 bis 6 steht

eingesetzt werden.

**5.** Verdickungsmittel gemäß Anspruch 1, **dadurch gekennzeichnet, dass** als Starter für die Komponente b) Verbindungen nachfolgender Struktur verwendet werden.

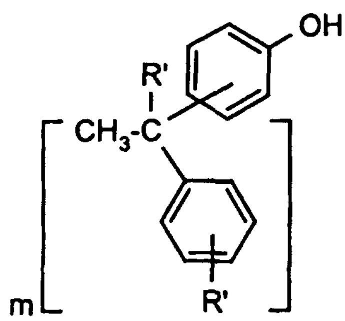

m = 0,5 bis 2,8 (statistische Mittelwerte)
R' = H, Methyl

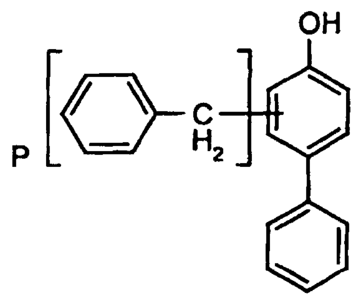

p = 0,5 bis 2,8
(m und p sind statistische Mittelwerte)

**6.** Verdickungsmittel nach Anspruch 1, **dadurch gekennzeichnet, dass** als Komponente c) Verbindungen der Formel

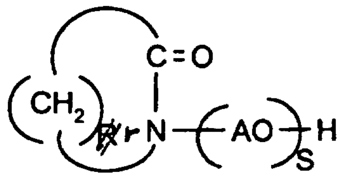

eingesetzt werden, worin

r eine Zahl von 2 bis 20 bedeutet,

AO einen Alkylenoxidrest mit 2 bis 3 Kohlenstoffatomen bedeutet und

s eine Zahl von 1 bis 30 bedeutet.

**7.** Verdickungsmittel nach Anspruch 1, **dadurch gekennzeichnet, dass** die Komponenten a) und die Summe der Komponenten b) und c) in einem Verhältnis von 3:1 bis 1:3 stehen.

**8.** Verdickungsmittel nach Anspruch 1, **dadurch gekennzeichnet, dass** die Komponenten b) und c) in einem Verhältnis von 2: 1 bis 1:8 stehen.

**9.** Verwendung der Verdickungsmittel-Zubereitung aus Anspruch 1 in wässrigen Systemen, ausgewählt aus der Gruppe bestehend aus wässrigen Kraftfahrzeugund Industrielacken, Putz- und Anstrichfarben, Druck- und Textilfarben, Pigmentdruckpasten, wässrigen pharmazeutischen, kosmetischen Formulierungen, Pflanzenschutz-formulierungen, Füllstoff- und Pigmentdispersionen, Zubereitungen von Waschmitteln, Klebstoffen, Wachsen und Polituren sowie für die Erdölförderung.

**Claims**

**1.** Thickener for thickening aqueous systems, comprising a mixture of

a) a water-soluble or dispersible thickener having urethane groups,

b) a non-ionic (optionally alk)aromatic emulsifier and

c) at least one further additive, and optionally

d) further auxiliary substances

**characterised in that** the component c) consists of at least one compound obtained by reacting $C_6$-$C_{22}$ aliphatic compounds containing active hydrogen atoms, with $C_2$-$C_4$ alkoxides.

**2.** Thickener according to claim 1, **characterised in that** as component c) compounds of the formula (I)

$$R_2\text{-}[(Q_2\text{-})_xH]_y \qquad (I)$$

are used,
wherein

$R_2$ denotes an optionally branched, and/or unsaturated aliphatic radical with 6 to 22 carbon atoms, and/or denotes a cycloaliphatic radical with 6 to 10 carbon atoms, and/or denotes a heterocyclic radical with 5 to 12, as is formed by removing active hydrogen from corresponding alcohols, amines, carboxylic acids and/or

amides,

$Q_2$ denotes alkylene oxide units, as are formed in the alkoxylation of hydroxyl group-containing starter molecules with $C_2$-$C_4$ alkylene oxides,

x is an integer from 1 to 30, and

y is an integer from 1 to 10.

**3.** Thickener according to claim 1, **characterised in that** as component b) compounds of the formula (II)

$$R\text{-}[O\text{-}(Q')_x\text{-}H]_y \quad (II)$$

in which

R denotes an aromatic and/or alkaromatic hydrocarbon radical with 6 to 50 carbon atoms optionally containing several aromatic rings and/or having inert substituents

Q' denotes alkylene oxide units, as are obtained in the alkoxylation of hydroxyl group-containing starter molecules with $C_2$-$C_4$ alkylene oxides,

x is an integer from 1 to 300, and

y is an integer from 1 to 20,

are used.

**4.** Thickener according to claim 1, **characterised in that** as component c) compounds of the general formula (III)

$$R_2\text{-}[EO_x\text{-}PO_y]\text{-}H \quad (III),$$

in which

$R_2$ denotes a linear radical with 6 to 15 carbon atoms, ethylene oxide, propylene oxide, ethylene oxide radicals and/or propylene oxide radicals,

x is an integer from 3 to 10,

y is an integer from 0 to 6,

are used.

**5.** Thickener according to claim 1, **characterised in that** as starter for the component b) compounds of the following structure are used:

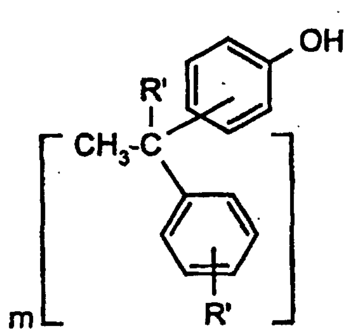

m = 0.5 to 2.8

(statistical mean values)

R' = H, methyl

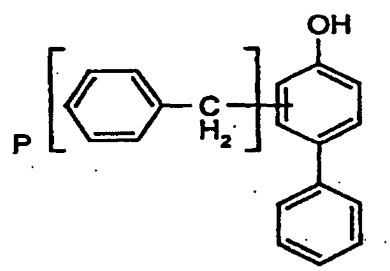

p = 0.5 to 2.8

(m and p are statistical mean values).

**6.** Thickener according to claim 1, **characterised in that** as component c) compounds of the formula

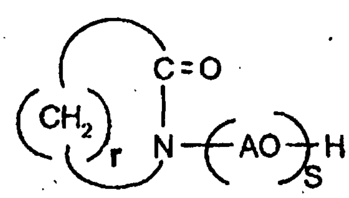

are used, wherein

r is an integer from 2 to 20,

AO denotes an alkylene oxide radical with 2 to 3 carbon atoms and

s is an integer from 1 to 30.

**7.** Thickener according to claim 1, **characterised in that** the components a) and the sum of the components b) and c) are in a ratio of 3 : 1 to 1 : 3.

**8.** Thickener according to claim 1, **characterised in that** the components b) and c) are in a ratio of 2 : 1 to 1 : 8.

9. Use of the thickener composition according to claim 1 in aqueous systems selected from the group comprising aqueous automotive and industrial paints, rendering agents and paints, printing inks and textile colorants, pigment printing pastes, aqueous pharmaceutical and cosmetic formulations, plant protection formulations, filler and pigment dispersions, detergents, adhesives, wax and polish formulations, as well as in petroleum extraction.

**Revendications**

1. Epaississant pour épaissir des systèmes aqueux, constitué d'un mélange de

   a) un épaississant soluble ou dispersible dans l'eau, présentant des groupes uréthanes,
   b) un émulsifiant non ionique aromatique (éventuellement alkylaromatique) et
   c) au moins un autre additif et éventuellement
   d) d'autres adjuvants

   **caractérisé en ce que** le composant c) est constitué d'au moins un composé qui est obtenu par réaction de composés aliphatiques de 6 à 22 atomes de C, contenant des atomes d'hydrogène actif, avec des alcokydes en $C_2$-$C_4$.

2. Epaississant selon la revendication 1, **caractérisé en ce qu'**on utilise comme composant c) des composés de formule (I)

$$R_2\text{-}[(Q_2\text{-})_xH]_y \qquad (I)$$

   dans laquelle

   $R_2$ est un radical aliphatique éventuellement ramifié et/ou insaturé avec 6 à 22 atomes de carbone, et/ou un radical cycloaliphatique avec 6 à 10 atomes de carbone, et/ou un radical hétérocyclique avec 5 à 12, tel qu'obtenu par séparation de l'hydrogène actif des alcools, amines, acides carboxyliques et/ou amides correspondants,
   $Q_2$ représente des unités oxyde d'alkylène, telles qu'obtenues par alcoxylation de molécules de départ contenant des groupes hydroxyle avec des oxydes d'alkylène en $C_2$-$C_4$,
   x représente un nombre de 1 à 30 et
   y représente un nombre de 1 à 10.

3. Epaississant selon la revendication 1, **caractérisé en ce qu'**on utilise comme composant b) un composé de formule (II)

$$R\text{-}[O\text{-}(Q')_x\text{-}H]_y \qquad (II),$$

   dans laquelle

   R représente un radical hydrocarboné de 6 à 50 atomes de carbone, aromatique et/ou alkaromatique qui peut éventuellement comprendre plusieurs cycles aromatiques et/ou présenter des substituants inertes,
   Q' représente des unités oxyde d'alkylène, telles qu'obtenues par alcoxylation de molécules de départ contenant des groupes hydroxyle avec des oxydes d'alkylène en $C_2$-$C_4$,
   x représente un nombre de 1 à 300 et
   y représente un nombre de 1 à 20.

4. Epaississant selon la revendication 1, **caractérisé en ce qu'**on utilise comme composant c) des composés de formule générale (III)

$$R_2\text{-}[EO_x\text{-}OPO_y]\text{-}H \qquad (III),$$

dans laquelle

$R_2$ représente un radical hydrocarboné linéaire avec 6 à 15 atomes de carbone, EO, PO signifient des radicaux oxyde d'éthylène respectivement oxyde de propylène,

x représente un nombre de 3 à 10,

y représente un nombre de 0 à 6.

5. Epaississant selon la revendication 1, **caractérisé en ce qu'**on utilise pour le composant b) des composés de structure suivante

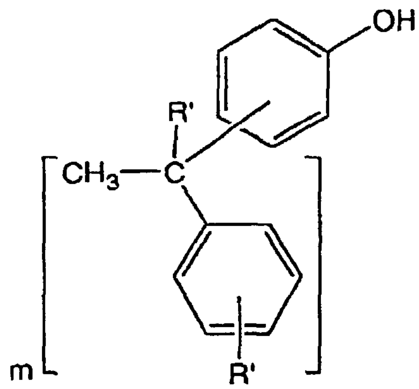

m = 0,5 à 2,8 (valeur moyenne statistique)
R' = H, méthyle

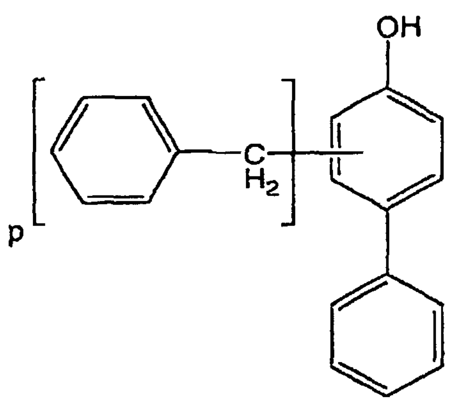

p = 0,5 à 2,8

(m et p sont des valeurs moyennes statistiques)

6. Epaississant selon la revendication 1, **caractérisé en ce qu'**on utilise comme composant c) des composés de formule

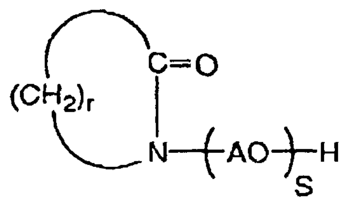

dans laquelle

r signifie un nombre de 2 à 20,
AO signifie un radical oxyde d'alkylène avec 2 à 3 atomes de carbone et
S signifie un nombre de 1 à 30.

**7.** Epaississant selon la revendication 1, **caractérisé en ce que** le composant a) et la somme des composants b) et c) sont dans un rapport de 3 : 1 à 1 : 3.

**8.** Epaississant selon la revendication 1, **caractérisé en ce que** les composants b) et c) sont dans un rapport de 2 : 1 à 1 : 8.

**9.** Utilisation de la préparation d'épaississant selon la revendication 1 dans des systèmes aqueux, choisis dans le groupe constitué par des laques pour automobiles et l'industrie, des couleurs pour enduits et peintures, des couleurs pour impression et pour textiles, des pâtes d'impression pigmentaires, des composés pharmaceutiques, des préparations cosmétiques, des formulations de protection de plantes, des dispersions de charges et de pigments, des préparations d'agents de lavage, des colles, des cires et des vernis ainsi que pour l'extraction du pétrole.